# FASCICULE DE BREVET EUROPEEN

(11) **EP 2 831 028 B1**
(45) Date de publication et mention de la délivrance du brevet: **04.05.2016**
(21) Numéro de dépôt: 13715345.8
(22) Date de dépôt: 18.03.2013
(51) Int. Cl.: C07C 59/285, C07C 51/34

(54) **NOUVELLE METHODE DE SYNTHESE DE L'ACIDE MUCIQUE PAR L'OZONE**
NEUES VERFAHREN ZUR SYNTHESE VON SCHLEIMSÄURE MITTELS OZON
NEW METHOD FOR SYNTHESISING MUCIC ACID USING OZONE

(30) Priorité: 27.03.2012 FR 1252715
(43) Date de publication de la demande: 04.02.2015
(73) Titulaire: Association de Gestion de l'Institut Polytechnique Lasalle Beauvais, 60000 Beauvais (FR)
(72) Inventeur: BONHOURE, Jean-Paul, 95390 Saint Prix (FR); ABBOUD, Hussein, 60000 Beauvais Cedex (FR); AUSSENAC, Thierry Robert, 60000 Beauvais (FR); COSTE, Christian Ulysse, 84870 Loriol Du Comtat (FR); HOANG, Levinh, 60000 Beauvais (FR); RALAINIRINA, Robert, 60000 Beauvais (FR); RANNOU, Alexis Christophe, 02000 Laon (FR)
(74) Mandataire: Hart-Davis, Jason
(86) Numéro de dépôt international: PCT/FR2013/050571
(87) Numéro de publication internationale: WO 2013/144483

(56) Documents cités:
- YUE-HWA YU ET AL: "Chemical oxidation of organic compounds by O2/UV", PROCEEDINGS 8TH OZONE WORLD CONGRESS, vol. 2, 1987, pages k61-k67, XP008158498,
- O.MARCQ ET AL: "Reaction pathways of glucose oxidation by ozone under acidic conditions", CARBOHYDRATE RESEARCH, vol. 344, no. 11, 2009, pages 1303-1309, XP002688448, PERGAMON ISSN: 0008-6215
- PH. NOMPEX ET AL: "Ozonation of selected molecules constituting cellular matter", OZONE, SCIENCE AND ENGINEERING, vol. 13, no. 3, 1991, pages 265-286, XP008158470,

## Description

La présente invention se rapporte à l'utilisation de l'ozone pour la synthèse de l'acide mucique. Un procédé de synthèse mettant en oeuvre l'ozone permet d'atteindre un rendement élevé en acide mucique, qui peut être obtenue sous forme cristalline, sans qu'il soit nécessaire d'employer ni solvant organique ni catalyseur, et avec une consommation d'énergie modérée.

### Etat de la technique

L'acide mucique (C₆H₁₀O₈; N°CAS526-99-8) présente la structure suivante :

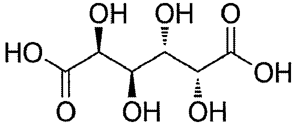

L'acide mucique est le plus souvent obtenu par oxydation du galactose ou de l'acide galacturonique ou des oligo- ou polysaccahrides contenant des unités galactose ou acide galacturonique.

L'acide mucique est un complexant puissant qui présente la propriété d'être totalement biodégradable. Il constitue donc une alternative de choix dans les formulations de produits lessiviels, dans certaines formulations de produits cosmétiques et, il peut servir de molécule plateforme dans l'industrie pharmaceutique.

Jusqu'à ce jour deux procédés sont utilisés pour la fabrication industrielle de l'acide mucique, il s'agit de :
- Un procédé purement chimique reposant sur l'utilisation comme agent oxydent de l'acide nitrique à concentration normale,
- Un procédé électrochimique classique mettant en oeuvre des électrodes graphite.

Il existe par ailleurs un procédé électrochimique mettant en oeuvre un intermédiaire réactionnel (le brome) lequel est régénéré *in situ* électro chimiquement. Ce dernier procédé est décrit dans l'article de Fauvarque et al., L'Actualité Chimique, octobre 1998, pages 48 à 50.

Les sources de matières premières sont également de deux sortes :
- l'acide galacturonique en solution aqueuse non purifiée,
- le galactose pur ou contenant des impuretés de type lactose, dulcite, quercite, ainsi que divers produits de condensation ou de polymérisation de type gomme.

Le document Yue-Hwa Yu et al: Chemical oxidation of organic compounds by O2/UV divulgue un procédé d'ozonation pour la préparation d'acide mucique à partir d'un composé différent.

Le procédé purement chimique utilisant l'acide nitrique à concentration normale présente comme inconvénient de générer des vapeurs nitreuses ainsi que des sous-produits nitrés difficiles à éliminer et présentant des dangers connus pour l'environnement. Un autre des inconvénients de cette méthode est la lenteur de la réaction. Par ailleurs, le procédé à l'acide nitrique génère d'abondantes quantités de mousse dont il est difficile de se séparer. La présence dans la solution mère aqueuse d'acide galacturonique, d'impuretés d'origine diverse génère, en l'absence de sélectivité de l'agent oxydant, des coproduits de réaction qui se retrouvent inclus dans les cristaux d'acide mucique obtenus en fin de procédé. Ceci implique que pour obtenir un produit quasi pur, il soit nécessaire de procéder à une dissolution des cristaux dans une solution exempte de contaminants et, après purification de la solution ainsi obtenue, de procéder à une nouvelle cristallisation. Il semble évident au travers des éléments exposés ci-dessus que le rendement de la réaction en est largement diminué. La réaction de l'acide nitrique sur l'acide galacturonique conduit à un rendement massique de la transformation aux environs de 60% M/M.

Le procédé électrochimique, lorsqu'il est utilisé dans sa version la plus simple (hors utilisation du brome), présente comme inconvénient d'être un procédé lent nécessitant un temps réactionnel extrêmement important. C'est un procédé très consommateur d'énergie dont les électrodes de graphite peuvent être considérées comme des agents consommables (i.e. disparition par usure des électrodes avec libération de sous-produits dans le milieu réactionnel). La pureté finale du produit synthétisé nécessite, comme dans la voie chimique, de procéder à la purification ultérieure des cristaux.

Le procédé électrochimique utilisant l'intermédiaire réactionnel brome, présente les mêmes inconvénients auxquels s'ajoutent les inconvénients connus de l'utilisation du brome ou de ses dérivés (risques industriels et impacts environnementaux connus). L'utilisation du brome dans cette variante du procédé électrochimique nécessite que cet intermédiaire réactionnel (brome) soit régénéré *in situ* par une méthode électrochimique, laquelle grève massivement la consommation d'énergie électrique et de ce fait l'économie du procédé.

### Résumé de l'invention

La présente invention se rapporte à une nouvelle méthode de synthèse par l'ozone de l'acide mucique et à un procédé de fabrication correspondant.

Selon un mode de réalisation, le produit de départ pour la synthèse de l'acide mucique est l'acide galacturonique. Dans un procédé préférentiel selon l'invention, le produit de départ est une solution mère aqueuse contenant l'acide galacturonique.

D'autres produits de départ peuvent cependant servir dans des synthèses par l'ozone de l'acide mucique. Des oligo- ou polysaccharides contenant des unités galactose peuvent être oxydés et hydrolysés (ou hydrolysé et ensuite oxydé) de façon à libérer in situ des unités monosaccharide d'acide galacturonique, ce dernier étant ensuite oxydé afin de former l'acide mucique.

Les solutions mères aqueuses qui peuvent être utilisées pour produire l'acide mucique par cette nouvelle méthode de synthèse par l'ozone, indépendamment de l'acide galacturonique, comprennent les suivantes : une solution de galactose pur ou contenant des impuretés de type lactose, dulcites, quercites, ainsi que divers produits de condensation ou de polymérisation qui peuvent se présenter sous forme de gommes. Toute solution mère aqueuse constituée de polysaccharides et/ou d'oligosaccharides d'origines végétales diverses contenant des unités galactose / acide galacturonique, et de concentrations massiques variables, peut être utilisée.

Dans un mode de réalisation préférentiel, la solution mère aqueuse est constituée d'acide galacturonique provenant généralement de la dégradation d'acides polygalacturoniques eux-mêmes issus de pectines d'origine végétale. De fait, la nature « biosourcée » des acides polygalacturoniques est assurée et garantie. On qualifie de produits « biosourcées » des produits dont l'origine végétale exclusive est garantie (confer notion de chimie verte du végétal).

La présente invention se rapporte donc dans un aspect à l'utilisation de l'ozone pour la synthèse de l'acide mucique à partir de mono-, oligo- et/ou polysaccharides comprenant des unités galactose ou acide galacturonique. De préférence, le produit de départ, comprenant des unités galactose ou acide galacturonique, comprend au moins choisi dans le groupe constitué par: l'acide galacturonique ; une solution de galactose pur ou contenant des impuretés de type lactose, dulcites, quercites, ainsi que divers produits de condensation ou de polymérisation qui peuvent se présenter sous forme de gommes ; polysaccharides et/ou d'oligosaccharides contenant des unités galactose et/ou acide galacturonique. De préférence, le produit de départ est présent sous forme de solution mère aqueuse. De manière particulièrement préférentielle, le produit de départ est une solution mère aqueuse d'acide galacturonique.

Selon un autre aspect, la présente invention se rapporte à un procédé de préparation de l'acide mucique comprenant les étapes suivantes :
(a) fourniture d'une solution mère aqueuse d'acide galacturonique;
(b) mise en contact de la solution mère aqueuse d'acide galacturonique fournie à l'étape (a) avec de l'ozone gazeux à une concentration comprise entre 150 et 220 g.m⁻³ TPN pour une durée d'au moins 50 minutes ;
(c) séparation de l'acide mucique formé à l'étape (b).

Dans ce procédé, la concentration en acide galacturonique dans la solution mère aqueuse fournie à l'étape (a) est de préférence d'au moins 8% en masse d'acide galacturonique soit 80 kg d'acide galacturonique pour 1000 kg de solution mère. La concentration maximale préférée en acide galacturonique est de 16% en masse d'acide galacturonique soit 160 kg d'acide galacturonique pour 1000 kg de solution mère. De manière plus préférentielle, la concentration en acide galacturonique dans la solution mère aqueuse sera comprise entre 11% et 13% en masse soit entre 110 et 130 kg d'acide galacturonique pour 1000 kg de solution mère.

Dans le procédé de l'invention, et notamment si on réalise une deuxième étape de traitement à l'ozone à une concentration inférieure à celle utilisée dans l'étape (b) mentionnée plus haut, la durée de l'étape (b), au cours de laquelle l'ozone est appliquée à une concentration d'au moins 150 g.m⁻³ TPN, est de préférence d'au plus 60 minutes.

Dans la présente invention, selon un mode de réalisation particulièrement préférentiel, postérieurement à l'étape (b), on réalise une deuxième phase de mise en contact de la solution réactionnelle avec l'ozone à une concentration comprise entre 90 et 120 g.m⁻³ TPN pour une durée comprise entre 300 et 380 minutes, préférentiellement entre 320 et 360 minutes.

Dans un mode avantageux de mise en oeuvre de la présenté invention, après traitement par l'ozone de la solution mère d'acide galacturonique, traitement qui s'effectue en une seule opération, de manière avantageuse comportant deux phases enchaînées, l'acide mucique précipite sous forme de cristaux d'acide mucique pur (pureté au-delà de 99%) dans un dispositif de séparation constitué par une décantation accélérée, comportant une zone de tranquillisation dans laquelle se concentrent les cristaux formés. Après extraction des cristaux de la zone de tranquillisation, ces cristaux peuvent être lavés puis séchés et conditionnés.

De manière avantageuse, dans le procédé de préparation de l'acide mucique selon l'invention, on n'utilise aucun solvant organique. On peut également et de manière appropriée n'employer aucun catalyseur homogène ni hétérogène durant les différentes phases de la réaction.

Le traitement à l'ozone de l'invention peut être réalisé de façon approprié à une température près de la température ambiante, soit entre 20 et 30°C.

Concernant la pression absolue dans le réacteur au cours de la synthèse de l'acide mucique selon la présente invention, la pression absolue sera avantageusement choisie entre 1,1 bar absolu et 2,5 bar absolu, et préférentiellement entre: 1,6 bar absolu et 2,2 bar absolu.

Dans le procédé de préparation de l'acide mucique de l'invention, l'ozone peut de manière appropriée être généré sur le gaz vecteur oxygène pur.

De manière préférentielle, l'acide mucique obtenu sous forme de cristaux dans le procédé de la présent invention est séparé de la solution aqueuse mère en cours de traitement par l'ozone et/ou après le traitement, par le moyen d'un décanteur de type accéléré. De préférence, l'acide mucique obtenu sous forme de cristaux est lavé avec de l'eau pure de type potable. De manière avantageuse, les cristaux d'acide mucique lavés sont ensuite soumis à un séchage à air chaud sec.

Dans un mode avantageux de mise en oeuvre de la présente invention, un procédé de synthèse par l'ozone de l'acide mucique laquelle synthèse peut s'effectuer en une seule opération comportant deux phases distinctes et comprenant les étapes constitutives suivantes :
(a) Fourniture de la solution mère d'acide galacturonique de pureté variable et dont le titre moyen est préférentiellement proche de 12% (M/M) (soit 120kg d'acide galacturonique pour 1000kg de solution mère) ;
(b) Introduction de la solution mère d'acide galacturonique à l'intérieur du procédé et validation des niveaux nécessaires au bon fonctionnement ;
(c) Mise en oeuvre de la recirculation de la solution mère sur la colonne d'équilibre et dans le réacteur et/ou simultanément sur le décanteur accéléré. Selon cette disposition, il y a création de deux boucles fonctionnant en parallèle qui alimentent le réacteur. La ou les boucles de recirculation étant établies, on procède à la mise en oeuvre simultanée de l'introduction du gaz ozoné à l'intérieur du réacteur (première phase de la synthèse par l'ozone à forte concentration, [O₃] concentration comprise entre 150 g.m⁻³ TPN et 220 g.m⁻³ TPN, préférentiellement aux environs de 160 à 170 g.m⁻³ TPN) ;
(d) Recirculation de la solution mère préalablement ozonée sur le décanteur pour séparer les cristaux natifs formés ; corrélativement mise hors circuit de la colonne d'équilibre et abaissement de la concentration d'ozone dans le gaz vecteur (deuxième phase de la synthèse par l'ozone à plus faible concentration, [O₃] concentration comprise entre 90 et 120 g.m⁻³ TPN, préférentiellement aux environs de 100 à 110 g.m⁻³ TPN) ; recirculation en service entre le réacteur et le décanteur ;
(e) Extraction des cristaux d'acide mucique et lavage de ces derniers dans le dispositif correspondant ;
(f) Extraction des cristaux d'acide mucique lavés et introduction de ces cristaux dans le dispositif de séchage ;
(g) Extraction des cristaux d'acide mucique séchés et admission vers le conditionnement final ;
(h) Durant l'ensemble des opérations d'ozonation, l'ozone résiduel présent après réaction est évacué vers une destruction thermique de l'ozone résiduel ou ce dernier est transformé en dioxygène avant rejet à l'atmosphère.

### Brève description des figures

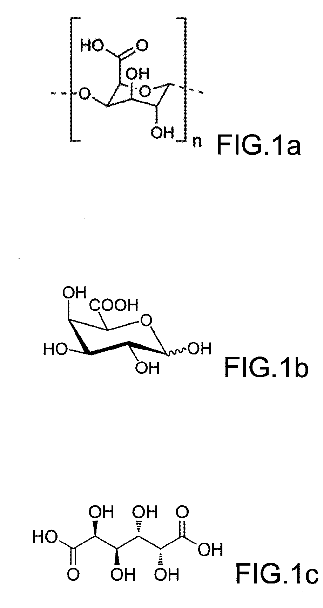

Les figures 1a, 1b et 1c représentent l'acide polygalacturonique, l'acide galacturonique et l'acide mucique respectivement.
La figure 2 représente de façon schématique un réacteur d'ozonation et sa colonne d'équilibre, ainsi que le décanteur accéléré, le dispositif de lavage des cristaux, le dispositif de séchage des cristaux qui conviennent pour la mise en oeuvre de la présente invention.

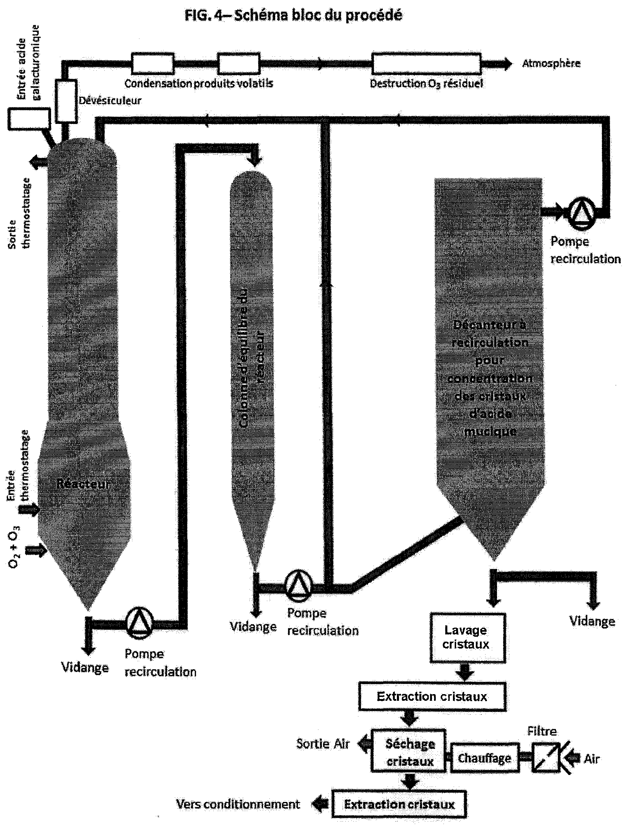

Les figures 3 et 4 représentent de façon schématique des manières avantageuses de mettre en oeuvre un procédé selon la présente invention.

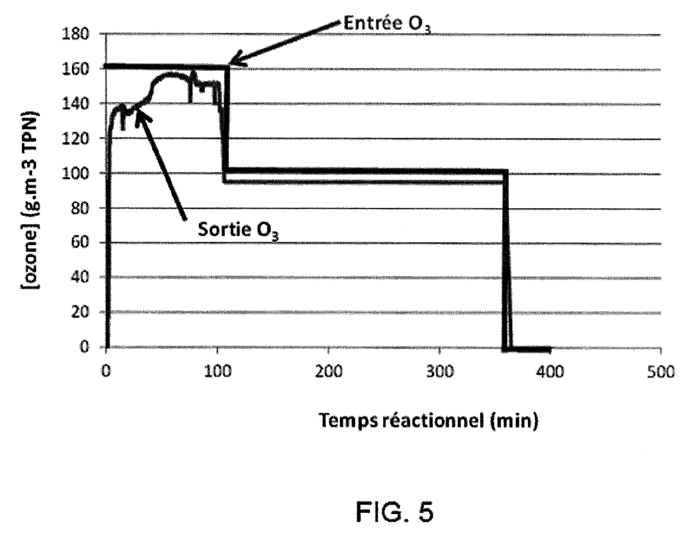

La figure 5 montre la cinétique réactionnelle complète des deux phases constitutives d'un procédé de synthèse selon l'invention.

### Description détaillée de l'invention

La présente invention concerne notamment l'utilisation de l'ozone et un procédé de mise en oeuvre de ce gaz réactif dans le but d'assurer la synthèse de l'acide mucique cristallisé à partir d'une solution mère aqueuse d'acide galacturonique. La solution mère aqueuse d'acide galacturonique provient généralement de la dégradation d'acides polygalacturoniques issu de pectines d'origine végétale. La solution mère aqueuse d'acide galacturonique à l'origine de la nouvelle méthode de synthèse de l'acide mucique peut être utilisé sans faire l'objet d'une purification préalable quelconque avant sa mise en oeuvre dans le procédé de l'invention.

Dans une perspective industrielle plus large, le produit servant de base à la fabrication de l'acide mucique peut avantageusement être l'acide polygalacturonique :

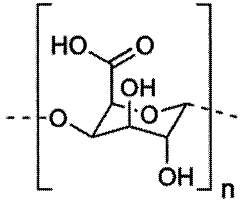

L'acide polygalacturonique est issu de la dégradation par hydrolyse (chimique ou enzymatique) des pectines d'origine végétale. C'est donc typiquement un produit « biosourcé ».

A partir de l'acide polygalacturonique, on fabrique habituellement l'acide galacturonique dont la représentation figure ci-dessous par coupure chimique ou enzymatique des liaisons inter motifs (liaisons intermoléculaires) de l'acide polygalacturonique.

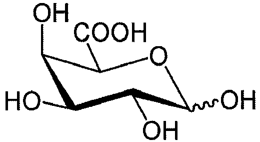

L'acide galacturonique étant issue d'un produit reconnue « biosourcé » (acide polygalacturonique), il est donc lui-même un produit « biosourcé ».

Dans le cadre de la présente invention, l'acide mucique : 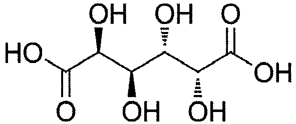 peut être synthétisé à partir de l'acide galacturonique mis en contact et en réaction avec l'ozone.

De manière avantageuse, l'ozone est fabriqué à partir d'oxygène liquide pur évaporé et de l'action de l'électricité utilisée sous forme de décharge de type couronne (Corona) ; l'ozone est donc un produit pur qui n'altère pas la qualité de produit bio-sourcé de l'acide mucique.

La demanderesse a mis en évidence qu'un traitement par l'ozone d'une solution aqueuse d'acide galacturonique, dans des conditions qui seront précisées dans les paragraphes qui suivent, permettait, en une seule opération unitaire, elle-même constituée de deux phases d'ozonation, d'obtenir des cristaux d'acide mucique d'une grande pureté et avec un rendement de transformation massique excellent - un rendement de transformation peut être observé entre 71% et 75% (M/M). De plus, ce nouveau procédé de fabrication conserve à l'acide mucique ainsi synthétisé sa qualification de produit bio-sourcé.

Comparé aux bilans énergétiques des solutions techniques actuelles, l'invention de la demanderesse présente un bilan énergétique très intéressant et très inférieur aux méthodes électrochimiques et chimiques. La consommation moyenne totale d'énergie électrique peut s'établir dans le cadre de la présente invention entre 9,1 kWh.kg⁻¹ d'acide mucique et 10,4 kWh.kg⁻¹ d'acide mucique.

Ceci s'explique de par le fait que le seul réactif mis en oeuvre est l'ozone, de par le fait également qu'il n'y a pas obligation de retraitement des cristaux en vue de leur purification, et enfin, de par le fait qu'il n'y a aucune nécessité de traiter des dégagements gazeux ou des effluents liquides fortement contaminés.

L'ozone utilisé pour cette synthèse chimique est avantageusement produit *in situ* à partir d'oxygène liquide de grande pureté, présentant un point de rosée de l'ordre de -86°C. Après évaporation de cet oxygène liquide et détente jusqu'à la pression requise (2,5 Bar), l'oxygène gazeux extrêmement sec est admis dans le générateur d'ozone à la sortie duquel on obtient le gaz vecteur « oxygène », supportant la quantité d'ozone choisie à la concentration choisie.

La demanderesse a mis en évidence que la synthèse de l'acide mucique à partir de l'acide galacturonique utilisant le réactif gazeux ozone, s'effectuait au mieux si une forte concentration en ozone est utilisé dans le gaz vecteur « oxygène ». De la même façon, la demanderesse a mis en évidence que cette synthèse s'effectue de préférence en deux phases, chacune des deux phases comprenant, selon un mode de réalisation optimale, l'utilisation d'une concentration d'ozone différente.

Lors de la première phase de la réaction la concentration d'ozone utilisée est de préférence très élevée, de l'ordre de 150 à 220 g.m⁻³ TPN, et la durée de la première phase de la réaction est de préférence de l'ordre de 60 à 100 minutes.

Dans le cadre du mode de réalisation préférentiel du procédé de l'invention en deux étapes, durant la première phase de la réaction, on peut faire circuler la solution aqueuse mère d'acide galacturonique en boucle fermée dans le réacteur et la colonne d'équilibre à l'aide de pompes assurant la continuité des débits. Dans le réacteur proprement dit, la solution aqueuse d'acide galaturonique parcourt alors le corps du réacteur dans le sens descendant (« down flow ») cependant que le gaz réactif « ozone » introduit à la partie basse du réacteur parcours ce dernier à contre-courant de la solution aqueuse d'acide galacturonique, dans le sens ascendant (« up flow »).

La réaction ozone sur acide galacturonique génère durant la première phase de la réaction des mousses de forte densité lesquelles mousses s'expansent dans la partie haute du réacteur où leur expansion est laissée libre. Ce lit de mousse n'est pas un inconvénient ou un frein à la réaction, il est au contraire une phase active de la réaction en favorisant les échanges entre le gaz ozoné et cette phase de mousse qui présente une surface spécifique développée extrêmement importante d'où une grande réactivité spécifique.

Au fil de la réaction, le lit de mousse diminue de hauteur, et cette diminution peut être favorisée par la recirculation de la solution mère d'acide galacturonique en cours de réaction et sa réinjection en tête de réacteur (partie haute du réacteur). En fin de première phase de la réaction, le lit de mousse ne présente plus alors qu'une hauteur de l'ordre de 0,1 à 0,2 m au-dessus de la surface liquide de la solution aqueuse mère.

La circulation de la solution aqueuse mère durant la réaction dans la colonne dite « d'équilibre » peut se faire de haut en bas sans introduction particulière d'ozone. La circulation de la solution aqueuse mère dans cette colonne ainsi que son sous tirage peut s'effectuer au moyen de pompes de circulation qui assurent la continuité du débit.

Selon un mode préférentiel d'une réaction avec l'ozone en deux phases, lors de la deuxième phase de la réaction, la demanderesse a mis en évidence qu'il était intéressant de poursuivre la dite réaction en diminuant la concentration de l'ozone dans le gaz vecteur ; concentration qui est réduite de 150 à 220 g.m⁻³ TPN à 90 à 120 g.m⁻³ TPN.

L'abaissement de la concentration d'ozone dans le gaz vecteur se justifie de par la poursuite normale de la réaction sans travailler avec un excès de réactif (ozone) ; ce qui correspond à un respect au plus juste de la stoechiométrie de la réaction. La deuxième phase de la réaction peut avantageusement se poursuivre durant un temps de l'ordre de 300 à 380 minutes.

Après le changement de la concentration d'ozone (deuxième phase de la réaction), la réaction de transformation se poursuit et la formation des germes de cristallisation s'accélèrent.

Les premiers germes de cristallisation (les premiers cristaux naissants) sont apparu durant la première phase de la réaction, (c'est-à-dire lorsque la réaction s'est produite à forte concentration d'ozone soit entre 150 et 220 g.m⁻³ TPN). Ces cristaux naissants apparaissent dans le soluté en cours de traitement après une durée de réaction de 50 à 60 minutes mesurée après le début de la réaction, durant la première phase.

Dès le changement de la concentration d'ozone, en début de deuxième phase de la réaction, les circuits assurant la recirculation de la solution aqueuse mère d'acide galacturonique peuvent être modifiés de façon très simple (ouverture et fermeture de trois vannes automatiques), de telle sorte que la colonne d'équilibre du réacteur soit mise hors circuit et que la boucle de recirculation de la solution aqueuse mère d'acide galacturonique s'établisse du réacteur vers le décanteur accéléré dans lequel seront retenus et renforcés les prémices des germes de cristallisation et, ultérieurement, les cristaux formés. A la sortie du décanteur accéléré, la solution aqueuse mère d'acide galacturonique peut être recirculée à l'aide de pompes vers le haut du réacteur où elle est réintroduite pour être mise en contact avec l'ozone suivant le principe de la boucle continue précédemment décrite.

Durant la phase finale de la réaction dans un mode préférentiel du procédé selon l'invention en deux étapes, la boucle de recirculation étant établie entre le réacteur et le décanteur, les cristaux se forment à l'intérieur du décanteur et y grossissent en se nourrissant de la solution aqueuse mère d'acide galacturonique préalablement ozonée.

Lorsque les cristaux à l'intérieur du décanteur ont atteint une taille critique (de l'ordre de 1,5 à 2,5 mm) qui correspond à une masse critique, ils décantent naturellement dans la zone de tranquillisation, et se concentrent en partie basse du cône du décanteur d'où ils peuvent être extraits séquentiellement en fonction du temps et du niveau atteint par les cristaux dans le cône situé en partie basse du décanteur.

Lors de l'extraction, les cristaux et une faible partie de la solution aqueuse mère d'acide galacturonique peuvent être évacués vers le dispositif de lavage des cristaux, lequel a pour but de séparer la faible partie de la solution aqueuse mère d'acide galacturonique, des cristaux, lesquels cristaux seront obtenus sans traces surfaciques de solution mère.

Le lavage des cristaux d'acide mucique formés et extraits de la partie basse du décanteur peut s'effectuer sans inconvénients, étant entendu et connu de l'homme de l'art que les cristaux d'acide mucique sont insolubles dans l'eau à température ambiante.

Le dispositif de lavage des cristaux d'acide mucique peut avantageusement être constitué d'une enceinte à l'intérieur de laquelle se trouve un tamis à mailles fines, réalisé en acier inoxydable. Le tamis peut avoir une forme générale de type trapézoïdale avec, en partie basse, une vis sans fin d'essorage et d'extraction permettant l'évacuation des cristaux lavés vers le dispositif de séchage. Le dispositif de lavage peut comporter, dans sa partie supérieure, au droit de l'arrivée des cristaux, des buses de pulvérisation alimentées en eau du réseau qui assurent le lavage des cristaux extraits avant essorage et extraction par la vis sans fin installée en partie basse. Les cristaux d'acide mucique lavés peuvent être extraits par la vis sans fin d'essorage et d'extraction vers le dispositif de séchage. Le dispositif de séchage peut comporter, en partie haute, une arrivée de cristaux humides convoyés par la vis sans fin d'extraction. Les cristaux peuvent être retenus dans le corps du sécheur par un tamis en acier inoxydable à mailles fines de forme trapézoïdale qui comporte, en partie basse, une vis sans fin d'extraction permettant d'extraire et de diriger les cristaux d'acide mucique séchés vers leur conditionnement. Le séchage peut s'effectuer à partir d'air atmosphérique filtré puis chauffé par une batterie de chauffage à résistances électriques. Le flux d'air chaud peut parcourir le corps du sécheur de bas en haut, il est alors canalisé depuis le dispositif de chauffage jusqu'au corps de séchage proprement dit par le tronc de pyramide inférieur du corps de séchage et traverse la couche de cristaux en attente de séchage. Le mouvement de l'air chaud peut être accéléré, dans sa phase accédante, par un dispositif constitué d'un ventilateur à forte dépression ou par une turbomachine capable de créer, dans le corps du sécheur, une dépression de quelques millimètres de colonne d'eau. Ce ventilateur ou cette turbomachine peut être installé en partie haute de l'enceinte de séchage et l'air chaud humide est évacué à l'atmosphère.

Le contrôle du séchage est de manière avantageuse placé sous la dépendance et le contrôle du facteur temps, une mesure du point de rosée de l'air de séchage en partie haute du sécheur, donne une indication utile du degré de séchage et permet une approche quantifiée de cette opération.

Une fois séchés les cristaux d'acide mucique peuvent être évacués de l'enceinte de séchage et dirigés vers le conditionnement final (emballage) à l'aide d'un dispositif de vis sans fin situé en partie basse de la zone trapézoïdale de rétention des cristaux dans le sécheur.

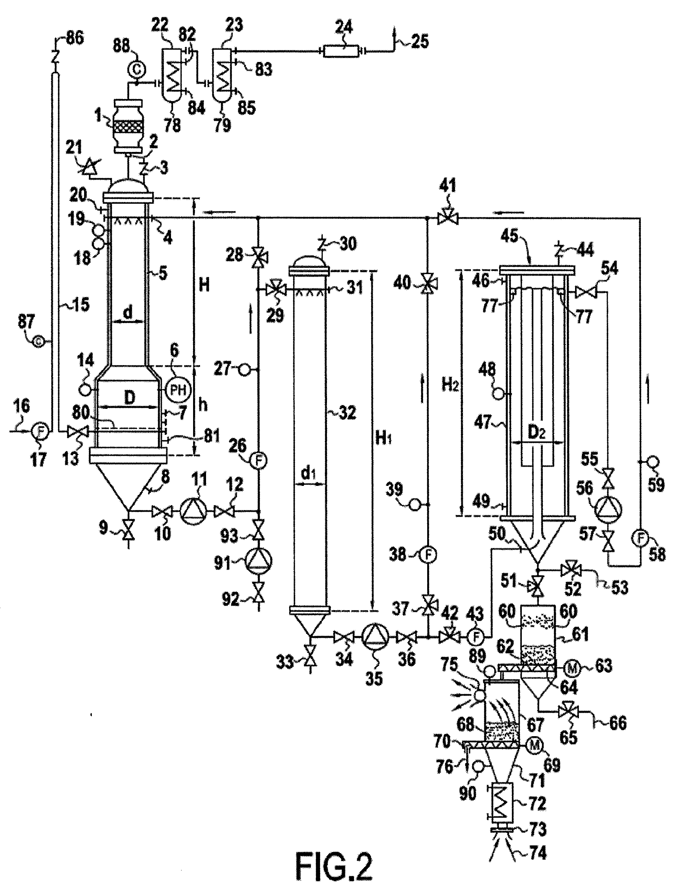

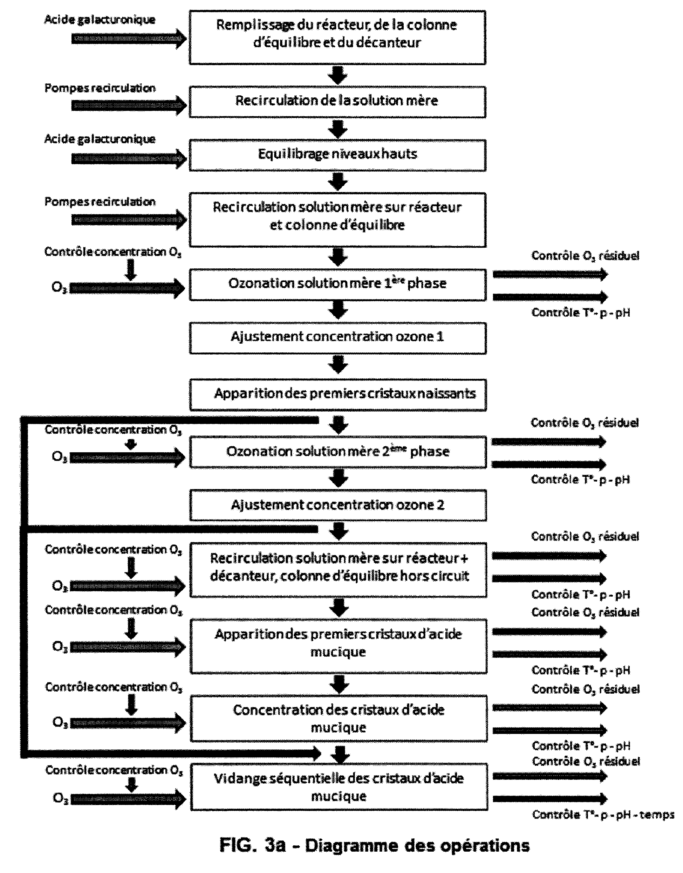

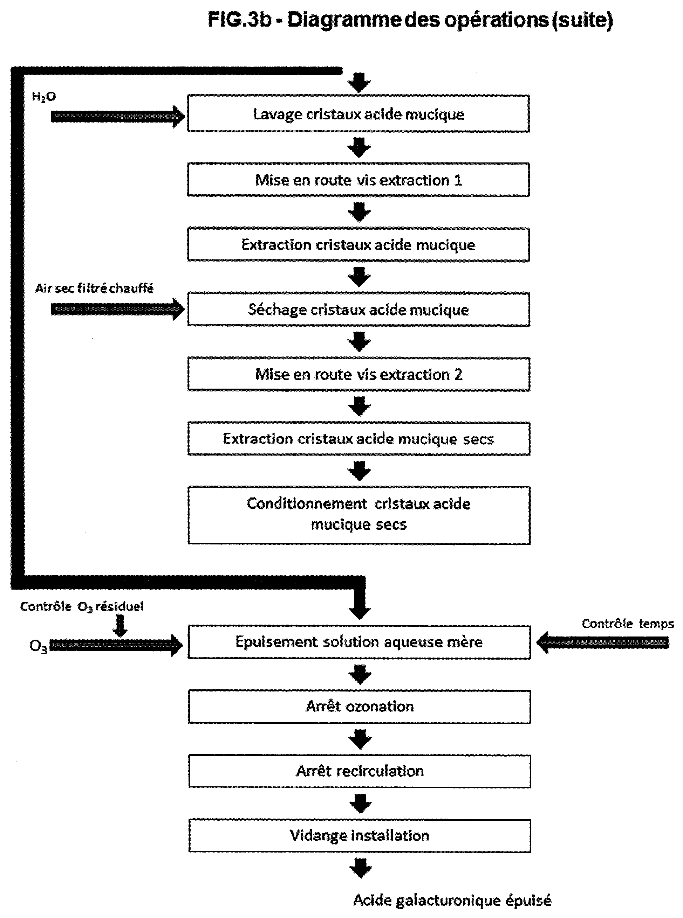

Dans un mode avantageux de mise en oeuvre de l'invention, le traitement de la solution aqueuse mère d'acide galacturonique traitée par l'ozone se fait suivant un procédé décrit par les figures 3 (diagramme des opérations) et 4 (schéma bloc du procédé) et des moyens généraux et de détail du procédé décrits dans la figure 2.

Les repères portés sur le schéma général de procédé de la figure 2 qui donne un exemple non limitatif du procédé qui convient pour la mise en oeuvre de la présente invention ; ces repères ont les identités suivantes :
- **1** dévésiculeur
- **2** orifice d'extraction de l'ozone résiduel
- **3** casse vide
- **4** retour de la recirculation de la solution mère d'acide galacturonique en boucle et dispositif d'introduction et de répartition de cette solution dans le réacteur
- **5** chemise d'eau (« water jacket »)
- **6** mesure de pH
- **7** hublot de visualisation de la réaction
- **8** prise d'échantillon
- **9** vidange du réacteur
- **10** vanne d'extraction de la solution mère d'acide galacturonique ozoné vers boucle de recirculation
- **11** pompe de recirculation
- **12** vanne de sectionnement de la boucle de recirculation
- **13** vanne de sectionnement de l'entrée d'oxygène ozoné
- **14** mesure de température du milieu réactionnel
- **15** lyre de dégardage de l'alimentation en oxygène ozone
- **16** arrivée d'oxygène ozoné
- **17** mesure de débit d'oxygène ozoné
- **18** mesure de température
- **19** mesure de pression
- **20** sortie du fluide de refroidissement du water jacket
- **21** soupape de sécurité
- **22** dispositif de condensation des volatils issus de la réaction
- **23** dispositif de condensation des volatils issus de la réaction
- **24** destruction de l'ozone résiduel en excès
- **25** mise à l'atmosphère
- **26** mesure de débit sur boucle de recirculation
- **27** mesure de pression dans boucle de recirculation
- **28** vanne automatique de sectionnement du circuit de la boucle de recirculation
- **29** vanne automatique de sectionnement à l'entrée de la colonne d'équilibre
- **30** casse vide de la colonne d'équilibre
- **31** dispositif de répartition de la solution mère d'acide galacturonique dans la colonne d'équilibre
- **32** colonne d'équilibre
- **33** vanne de vidange de la colonne d'équilibre
- **34** vanne de sectionnement de la boucle de recirculation entre la colonne d'équilibre et le décanteur accéléré
- **35** pompe de recirculation
- **36** vanne de sectionnement
- **37** vanne automatique de sectionnement sur boucle de recirculation après colonne d'équilibre
- **38** mesure de débit dans la boucle de recirculation
- **39** mesure de pression dans la boucle de recirculation
- **40** vanne automatique de sectionnement de la boucle de recirculation
- **41** vanne automatique de sectionnement du retour du décanteur accéléré
- **42** vanne automatique de sectionnement à l'entrée du décanteur pour établir la boucle de recirculation sur le décanteur
- **43** mesure de débit de recirculation sur le décanteur
- **44** casse vide sur le décanteur
- **45** sortie ozone résiduel vers destruction
- **46** sortie fluide de water jacket
- **47** water jacket du décanteur accéléré
- **48** mesure de température dans le décanteur
- **49** entrée fluide dans le water jacket
- **50** entrée solution mère d'acide galacturonique en recirculation sur décanteur
- **51** vanne automatique d'extraction des cristaux d'acide mucique
- **52** vanne automatique de sectionnement du fluide de rinçage
- **53** arrivée de fluide de rinçage
- **54** vanne de sectionnement de la sortie du décanteur
- **55** vanne de sectionnement du circuit de recirculation décanteur
- **56** pompe de recirculation du circuit décanteur/réacteur
- **57** vanne de sectionnement de la pompe de recirculation
- **58** mesure de débit de la boucle de recirculation décanteur/réacteur
- **59** mesure de pression
- **60** arrivées eau de lavage des cristaux d'acide mucique et pulvérisation
- **61** corps de laveur de cristaux
- **62** masse de cristaux en cours de lavage
- **63** moteur d'entrainement de la vis d'extraction des cristaux lavés
- **64** vis d'extraction
- **65** vanne automatique de vidange de l'eau de lavage
- **66** évacuation de l'eau de lavage
- **67** corps de sécheur de cristaux
- **68** masse de cristaux en cours de séchage
- **69** moteur d'entrainement de la vis d'extraction de cristaux séchés
- **70** vis d'extraction des cristaux séchés
- **71** tronc de pyramide de diffusion d'air sec et chaud
- **72** corps de chauffe de l'air de séchage
- **73** filtre d'air de séchage
- **74** entrée d'air de séchage
- **75** machine d'aspiration de l'air de séchage
- **76** sortie de cristaux vers conditionnement
- **77** goulotte de reprise de la solution mère d'acide galacturonique pour recirculation vers réacteur
- **78** sortie des condensables
- **79** sortie des condensables
- **80** dispositifs d'injection de gaz ozoné dans le réacteur
- **81** entrée de fluide de refroidissement dans le water jacket
- **82** entrée fluide de refroidissement sur condenseur des volatils
- **83** entrée fluide de refroidissement sur condenseur des volatils
- **84** sortie fluide de refroidissement sur condenseur des volatils
- **85** sortie fluide de refroidissement sur condenseur des volatils
- **86** dispositif de casse vide en partie haute de lyre de dégardage
- **87** dispositif de mesure de la concentration en ozone dans le gaz vecteur en entrée de procédé
- **88** dispositif de mesure de la concentration en ozone résiduel en sortie de réacteur
- **89** dispositif de mesure du point de rosée de l'air chaud en sortie de sécheur de cristaux
- **90** mesure de température de l'air de séchage des cristaux
- **91** pompe de remplissage du procédé
- **92** orifice de remplissage du procédé
- **93** vanne de sectionnement
- **D** diamètre de la partie basse du réacteur
- **d** diamètre de la partie haute du réacteur (zone d'expansion des mousses)
- **H** hauteur de la colonne d'expansion
- **h** hauteur de la partie basse du réacteur
- **d1** diamètre de la colonne d'équilibre
- **H1** hauteur de la colonne d'équilibre
- **D2** diamètre du décanteur
- **H2** hauteur cylindrique du décanteur

Le début de l'opération de synthèse de l'acide mucique à partir de l'acide galacturonique commence de façon appropriée par le remplissage de l'installation aux niveaux requis. Les trois dispositifs principaux, le réacteur, la colonne d'équilibre, le décanteur accéléré, sont de façon appropriée remplis de solution aqueuse d'acide galacturonique, par l'intermédiaire de l'orifice de remplissage (92). Une pompe (91) peut être utilisée afin de propulser la solution d'acide galacturonique dans l'ensemble des trois éléments et établir les niveaux de fonctionnement requis. Pour ajuster finement les niveaux de remplissage il est possible de jouer sur les débits de la pompe (11), de la pompe (35), et de la pompe (56).

De manière avantageuse, une fois les débits requis validés dans les trois dispositifs principaux, le procédé d'ozonation peut démarrer.

Compte tenu des concentrations en ozone dans le gaz vecteur préférables pour la synthèse de l'acide mucique (150 à 220 g.m⁻³ TPN) durant la première phase et (90 à 120 g.m⁻³ TPN) durant la seconde phase, le gaz vecteur préféré pour la génération de l'ozone est de l'oxygène pur et sec issu de l'évaporation d'oxygène liquide. Il est en effet difficile d'atteindre les concentrations citées précédemment avec un autre gaz vecteur.

Le gaz vecteur oxygène supportant l'ozone à forte concentration peut être introduit dans le réacteur par l'intermédiaire de l'arrivée (16) et le débit contrôlé par le dispositif de mesure de débit (17). La concentration d'ozone dans le gaz vecteur peut être mesurée par l'analyseur (87). Avant son introduction dans le réacteur, le gaz ozoné parcourt de manière avantageuse la lyre de dégardage (15), l'introduction proprement dite dans le réacteur s'effectuant par la vanne de sectionnement (13) qui alimente le dispositif interne de diffusion (de bullage) (80). Le dispositif de bullage est avantageusement constitué par des disques poreux montés sur support capables de créer des bulles de taille comprise entre 2 et 4 mm. Le gaz ozoné transformé en bulles traverse la masse de solution d'acide galacturonique en cours de réaction. Dès l'établissement du flux de gaz ozoné, dès que les bulles traversent la solution d'acide galacturonique, il se créé à l'interface liquide des mousses denses qui s'expansent dans la partie haute du réacteur ; c'est-à-dire dans la partie matérialisée par le diamètre (d) et la hauteur libre (H). Ces mousses denses constituent un système réactionnel complexe qui participe activement à l'accomplissement de la cinétique réactionnelle de transformation de l'acide galacturonique en acide mucique. En effet, les mousses présentent une surface spécifique développée extrêmement importante, qui favorise l'échange et le transfert gazeux entre le gaz ozoné à très forte concentration et la phase liquide d'acide galacturonique.

Comme il a été dit plus haut, au cours de la synthèse de l'acide mucique selon la présente invention, la pression absolue sera avantageusement choisie entre 1,1 bar absolu et 2,5 bar absolu, et préférentiellement entre: 1,6 bar absolu et 2,2 bar absolu. Les effets de la pression dans le réacteur peuvent notamment comprendre : un meilleur transfert de l'ozone de la phase gazeuse à la phase liquide en diminuant la résistance de transfert à l'interface gaz/liquide et une meilleure alimentation en ozone du milieu réactionnel. La réaction côté liquide est une réaction semi-rapide qui ne consomme pas instantanément l'ozone transféré, et il est donc souhaitable de rompre la tendance à l'équilibre à l'interface et forcer le transfert dans le sens gaz/liquide et de ce fait alimenter en permanence le phénomène de diffusivité côté liquide. Par ailleurs, la pression d'application dans le réacteur peut améliorer de façon marquée le transfert de l'ozone dans la phase mousse qui est une phase très active avec une surface spécifique réactionnelle extrêmement développée donc très active dans la synthèse.

Durant la phase d'ozonation de la solution d'acide galacturonique, cette dernière recircule entre le réacteur et la colonne d'équilibre par l'intermédiaire de la pompe (11), puis de la colonne d'équilibre vers le réacteur ou, de la colonne d'équilibre vers le décanteur accéléré ou, de la colonne d'équilibre vers le réacteur et, du décanteur accéléré vers le réacteur. Les deux recirculations constituent des boucles de recirculation parallèles qui sont mises en oeuvre par l'intermédiaire de la pompe (35) et de la mesure de débit (38) pour ce qui est de la boucle courte (sortie colonne d'équilibre - réacteur) et par l'intermédiaire de la pompe (35) et de la mesure de débit (43) ainsi que de la pompe (56) pour ce qui est la boucle longue (sortie colonne d'équilibre - décanteur accéléré).

Dans le cas de l'utilisation de la boucle courte, sortie de la colonne d'équilibre - réacteur, la totalité du débit de recirculation généré par la pompe (35), passe au travers de la mesure de débit (38) et par l'intermédiaire des vannes automatiques (37 et 40), et rejoint le haut du réacteur où la solution d'acide galacturonique en cours de traitement est admise dans le réacteur par le dispositif (4).

Dans le cas de l'utilisation de la boucle courte et de la boucle longue en parallèle, le débit sortant de la colonne d'équilibre est propulsé par la pompe (35) et partialisé dans le circuit court dont le débit est mesuré par le dispositif (38) et dans le circuit long dont le débit est mesuré par le dispositif (43). L'équilibre entre les deux débits (boucle courte et boucle longue) est réglé par les mesures de débit (38 et 43).

Sur la boucle longue en sortie de décanteur accéléré se trouve la pompe (56) et le dispositif de mesure de débit (58) qui permettent le retour de la solution aqueuse d'acide galacturonique en cours de traitement vers le dispositif de réintroduction dans le réacteur (4).

Dans le cas de l'utilisation de la boucle longue exclusivement, c'est-à-dire durant la phase où les cristaux d'acide mucique sont décantés dans le décanteur accéléré, la boucle courte en sortie de colonne d'équilibre est neutralisée (vannes 37 et 40 fermées), et le débit de la solution d'acide galacturonique propulsée par la pompe (35) rejoint l'entrée du décanteur accéléré (50) par l'intermédiaire de la vanne automatique de sectionnement (42) et le dispositif de mesure de débit (43). En sortie de la goulotte de reprise supérieure du décanteur accéléré (77), la solution d'acide galacturonique en cours de traitement est reprise et accélérée par la pompe (56) dont le débit est mesuré par le dispositif (58) et dirigé au travers de la vanne automatique (41) vers le dispositif d'introduction dans le réacteur (4). En partie supérieure du réacteur, le gaz vecteur oxygène plus l'ozone résiduel est évacué vers un dispositif de destruction thermique de l'ozone résiduel en excès (24) avant rejet à l'atmosphère par le dispositif (25).

Dans ce mode de réalisation préférentiel décrit à la Figure 2, en sortie immédiate du réacteur, sur sa partie supérieure, se trouve monté un dispositif dévésiculeur (1), capable de séparer les fines gouttelettes entraînées par le gaz vecteur. Les fines gouttelettes coalescées, retournent dans le réacteur cependant que le gaz vecteur traverse le dispositif de mesure de concentration (88), mesurant la concentration d'ozone résiduel après réaction. Le gaz vecteur poursuit son cheminement vers la destruction d'ozone (24) en traversant deux dispositifs de condensation des volatils montés en série (22 et 23). Ces dispositifs sont alimentés en eau glacée par l'intermédiaire des entrées et sorties repérées (82, 83, 84 et 85).

La partie supérieure du réacteur comporte les dispositifs suivants :
- Une soupape de sécurité tarée (21) destinée à éviter toute surpression dans le réacteur ;
- Un dispositif casse vide (3) destiné à permettre une vidange aisée du réacteur.

Le corps du réacteur proprement dit comporte avantageusement des mesures de température et de pression (18 et 19 respectivement). Le corps cylindrique inférieur du réacteur ainsi que le corps cylindrique supérieur du réacteur sont avantageusement munis d'un dispositif de thermorégulation constitué par un water jacket alimenté par les entrées (81) et la sortie (20). La partie inférieure du réacteur (sommet de la partie conique) est avantageusement munie d'un dispositif de vidange (9). Un dispositif (8) permet le prélèvement d'échantillons de la solution en réaction. Dans un mode de réalisation, la colonne d'équilibre (32) est principalement constituée d'un corps cylindrique muni à la partie supérieure d'un fonds bombé équipé d'un dispositif casse vide (30). Un dispositif (31) peut permettre l'introduction à la partie supérieure de la solution d'acide galacturonique en réaction qui parcourt la colonne d'équilibre de haut en bas, c'est-à-dire en « down flow ». La partie inférieure de la colonne d'équilibre peut être munie d'un cône portant à son sommet un dispositif de vidange (33) ainsi qu'un départ de soluté en recirculation (34).

Dans ce mode de réalisation préférentiel décrit à la Figure 2, le décanteur accéléré destiné à favoriser la cristallisation de l'acide mucique, la concentration des cristaux et leur évacuation vers le dispositif de lavage, est avantageusement constitué par un corps cylindrique fermé en partie supérieure par un fonds plat muni d'un dispositif (45) d'évacuation d'ozone résiduel vers la destruction. Ce même fonds plat est muni d'un dispositif casse vide (44). Le corps cylindrique du décanteur accéléré dans ce mode de réalisation comporte un dispositif de thermorégulation constitué par un water jacket (47) alimenté par l'entrée (49) et la sortie (46). Un dispositif de mesure de la température (48) permet le contrôle de la température interne du décanteur. La partie basse du décanteur est fermée par un fonds conique destinée à la recirculation, à la concentration et au stockage des cristaux formés et nourris durant la phase de décantation. Le sommet du cône est muni d'une vanne automatique d'extraction des cristaux (51) ainsi que d'un dispositif de nettoyage à l'eau claire constituée d'une arrivée d'eau claire (53) et d'une vanne automatique (52). A l'intérieur du décanteur accéléré se trouve une cheminée de forme cylindro-conique permettant le mélange intime du soluté en réaction et des germes de cristallisation naissants contenus dans la partie basse du cône. Ce mélange est refoulé sous la pression hydrodynamique fournie par la pompe (35) vers le sommet du décanteur où le flux se stabilise avant de redescendre dans la chemise qui canalise le mélange vers le bas du décanteur ; outre le fait de canaliser le mélange soluté-germes de cristallisation, cette chemise a pour but de séparer physiquement la zone de descente de la zone de remontée dite zone de décantation. Cette zone de remontée est la zone annulaire comprise entre le corps extérieur cylindrique du décanteur accéléré et la partie extérieure de la chemise de canalisation du mélange. Dans cette zone de tranquillisation, le flux de soluté se sépare de façon continue des cristaux en formation qui chutent vers le bas du décanteur.

Dans ce mode de réalisation préférentiel décrite à la Figure 2, à la partie supérieure de la zone de décantation se trouve une goulotte de reprise (77) qui recueille le soluté exempt de cristaux et l'achemine vers la pompe de recirculation (56). A la sortie de la vanne automatique (51) dont le fonctionnement est cyclique et réglable, les cristaux concentrés en partie basse du cône du décanteur accéléré, sont évacués vers un dispositif de lavage (61) muni d'arrivées multiples d'eau de lavage (60). Les cristaux peuvent être stockés dans une partie trapézoïdale du dispositif (62) et évacués par une vis sans fin (64) motorisée (63). L'eau de lavage des cristaux d'acide mucique peut être recueillie en partie basse du dispositif de lavage dans une zone pyramidale d'où elle est évacuée en partie basse par une vanne automatique (65) et une mise à l'égout (66). A la sortie de la vis sans fin (64), les cristaux d'acide mucique synthétisés peuvent être admis dans un dispositif de séchage (67) de forme trapézoïdale, où ils sont stockés (68) et le lit de cristaux parcouru de bas en haut par un flux d'air chaud canalisé par la partie pyramidale (71). L'air chaud peut être généré à partir d'un dispositif de chauffage (72) muni de résistances électriques après avoir été filtré par un dispositif fin de filtration (73) où il est canalisé par le dispositif d'entrée (74). La température de l'air chaud peut être mesurée par un dispositif de mesure de température (90) situé sur la partie pyramidale de canalisation de l'air chaud. Le mouvement de l'air chaud peut être accéléré par un dispositif (75) qui peut être indifféremment un ventilateur à forte dépression ou une turbo machine capable de créer une dépression de quelques millimètres de colonne d'eau.

Le séchage des cristaux d'acide mucique synthétisés est sous la dépendance de deux paramètres qui sont respectivement la température (90) et le point de rosée (89). Une fois séchés, les cristaux peuvent être évacués par la vis sans fin (70) entrainée par le moteur (69). En sortie de la vis sans fin (76), les cristaux secs d'acide mucique synthétisés sont disponibles pour conditionnement.

### Résultats obtenus

L'ensemble des conditions opératoires préférentiels identifiés et des résultats obtenus sont présentés dans le tableau I ci-après.

**Tableau I**

| **Synthèse des conditions opératoires et des résultats obtenus** | |
|---|---|
| **Rubriques** | **Valeurs** |
| Concentration en acide galacturonique de la solution mère (C_{M}) | C_{M} ≥ 120 g.L⁻¹ |
| Nombre d'opérations chimiques de la synthèse | 1 |
| Nombre de phases enchaînées de la synthèse | 2 |
| Nombre d'opérations enchaînées de la synthèse | 4 |
| Gaz vecteur utilisé / pureté | O₂/99% |
| Concentration d'ozone (phase 1) (C₁) | C₁ = 160 / 170 g.m⁻³ TPN |
| Concentration d'ozone (phase 2) (C₂) | C₂ = 100 / 110 g.m⁻³ TPN |
| Temps réactionnel (phase 1) (T₁) | 50 min<T₁<60 min |
| Temps réactionnel (phase 2) (T₂) | 300 min<T₂<380 min |
| Temps réactionnel total (T_{T}) | T_{T}=420 min |
| Température de la réaction (t) | 20°C<t<30°C |
| Rendement de la réaction chimique de transformation (acide mucique formé et cristallisé) (η) | 71%(M/M)<n<75%(M/M) |
| Pureté de l'acide mucique cristallisé (P) | P > 99% |
| Energie totale consommée pour la synthèse de l'acide mucique cristallisé (E) | 9,1 KWh.kg⁻¹<E<10,4 KWh.kg⁻¹ |

La lecture du tableau I ci-dessus fait apparaître la qualité de la découverte faite par la demanderesse. Comparé aux techniques classiquement mises en oeuvre [procédé chimique reposant sur utilisation de l'acide nitrique ou, procédé électrochimique à électrode graphite ou, procédé électrochimique avec intermédiaire réactionnel (brome)], le procédé découvert par la demanderesse (synthèse d'acide mucique par ozonation), ne présente aucun dégagement gazeux (i.e. vapeurs nitreuses pour la solution chimique, CO ou CO₂ pour les solutions électrochimiques), n'est pas consommateur d'électrodes graphite, ne nécessite pas la repurification des cristaux obtenus, ne nécessite pas le traitement de gaz nocifs avant rejet à l'atmosphère.

L'énergie totale consommée par la synthèse pour aboutir à la production de cristaux d'acide mucique de haute pureté, ne nécessite que 9,1 à 10,4 KWh.kg⁻¹, comparé aux 30 à 40 KWh.kg⁻¹ nécessaires pour la synthèse par voie électrochimique par exemple.

Enfin, cette nouvelle voie de synthèse de l'acide mucique est une voie propre et sobre, respectueuse de l'environnement et valorisant des matières premières totalement biosourcées.

### Paramètres préférés de l'invention

De façon générale, il a été observé et constaté que les paramètres qui suivent conduisent à des résultats optimaux en termes de transformation de l'acide galacturonique en acide mucique cristallisé :
- Le rapport caractéristique entre la hauteur (h) de la partie basse du réacteur et le diamètre (D) de cette même partie sera avantageusement compris entre 1 et 1,7 D ;
- Le rapport caractéristique de la hauteur du cône de raccordement de la partie basse de diamètre (D) à la partie haute de diamètre (d) sera avantageusement compris entre 0,3 et 0,7 D ;
- Le rapport caractéristique de la hauteur de la colonne supérieure du réacteur (H) au diamètre de cette même colonne (d) sera avantageusement compris entre 4 et 6 d ;
- Le rapport caractéristique entre le diamètre de la partie basse du réacteur (D) et la hauteur totale de la colonne (H+h) sera avantageusement compris entre 3 et 5 D ;
- La hauteur du cône bas fermant la partie basse du réacteur de diamètre (D) sera avantageusement comprise entre 0,5 et 1 D ;
- Le rapport caractéristique de la hauteur (H1) de la colonne d'équilibre par rapport à son diamètre (d1) sera avantageusement compris entre 12 et 17 d1 ;
- Le rapport caractéristique de la hauteur (H2) de la partie cylindrique du décanteur accéléré à son diamètre (D2) sera avantageusement compris entre 2,4 et 3,0 D2 ;
- Le rapport caractéristique du diamètre de la chemise interne du décanteur accéléré au diamètre (D2) du même décanteur accéléré sera avantageusement compris entre 0,3 et 0,7 D2.
- L'intérieur du réacteur, l'intérieur de la colonne d'équilibre et l'intérieur du décanteur accéléré peuvent être avantageusement munis de dispositifs de lavage constitués par des buses ou des boules de lavages connues de l'homme de l'art et alimentées par de l'eau sous pression (53).

## Revendications

1. Utilisation de l'ozone pour la synthèse de l'acide mucique à partir de mono-, oligo- et/ou polysaccharides comprenant des unités galactose ou acide galacturonique.

2. Utilisation selon la revendication 1, selon laquelle le produit de départ comprend au moins choisi dans le groupe constitué par: l'acide galacturonique ; une solution de galactose pur ou contenant des impuretés de type lactose, dulcites, quercites, ainsi que divers produits de condensation ou de polymérisation qui peuvent se présenter sous forme de gommes ; polysaccharides et/ou d'oligosaccharides contenant des unités galactose et/ou acide galacturonique.

3. Utilisation selon la revendication 1 ou 2, selon laquelle le produit de départ est présent sous forme de solution mère aqueuse.

4. Utilisation selon la revendication 3, selon laquelle le produit de départ est une solution mère aqueuse d'acide galacturonique.

5. Procédé de préparation de l'acide mucique comprenant les étapes suivantes :
(a) fourniture d'une solution mère aqueuse d'acide galacturonique;
(b) mise en contact de la solution mère aqueuse d'acide galacturonique fournie à l'étape (a) avec de l'ozone gazeux à une concentration comprise entre 150 et 220 g.m⁻³ TPN pour une durée d'au moins 50 minutes ;
(c) séparation de l'acide mucique formé à l'étape (b).

6. Procédé selon la revendication 5, selon lequel la concentration en acide galacturonique dans la solution mère aqueuse fournie à l'étape (a) est d'au moins 8% en masse d'acide galacturonique soit 80 kg d'acide galacturonique pour 1000 kg de solution mère.

7. Procédé selon la revendication 6, selon lequel la durée de l'étape (b), la mise en contact avec de l'ozone, est d'au plus 60 minutes.

8. Procédé de préparation de l'acide mucique selon l'une quelconque des revendications 5 à 7, selon lequel, postérieurement à l'étape (b), on réalise une deuxième phase de mise en contact de la solution réactionnelle avec l'ozone à une concentration comprise entre 90 et 120 g.m⁻³ TPN pour une durée comprise entre 300 et 380 minutes, préférentiellement entre 320 et 360 minutes.

9. Procédé de préparation de l'acide mucique selon l'une quelconque des revendications 5 à 8, dans lequel on n'utilise aucun solvant organique.

10. Procédé de préparation de l'acide mucique selon l'une quelconque des revendications 5 à 9, dans lequel on ne met en oeuvre aucun catalyseur homogène ou hétérogène durant les différentes phases de la réaction.

11. Procédé de préparation de l'acide mucique selon l'une quelconque des revendications 5 à 10, dans lequel l'ozone est généré sur le gaz vecteur oxygène pur.

12. Procédé de préparation de l'acide mucique selon l'une quelconque des revendications 5 à 11, dans lequel l'acide mucique obtenu sous forme de cristaux est séparé de la solution aqueuse mère en cours de traitement par l'ozone et/ou après le traitement, par le moyen d'un décanteur de type accéléré.

13. Procédé de préparation de l'acide mucique selon l'une quelconque des revendications 5 à 12, dans lequel l'acide mucique obtenu sous forme de cristaux est lavé avec de l'eau pure de type potable.

14. Procédé de préparation de l'acide mucique selon la revendication 13, dans lequel les cristaux d'acide mucique lavés sont ensuite soumis à un séchage à air chaud sec.

## Patentansprüche

1. Verwendung von Ozon für die Synthese von Schleimsäure aus Mono-, Oligo- und/oder Polysacchariden, die Galactose-Einheiten oder Galacturonsäure umfassen.

2. Verwendung nach Anspruch 1, wonach das Ausgangsprodukt wenigstens umfasst, ausgewählt aus der Gruppe bestehend aus: Galacturonsäure; einer Lösung aus reiner Galactose oder enthaltend Unreinheiten vom Typ Lactose, Dulcite, Quercite, sowie verschiedene Kondensations- oder Polymerisationsprodukte, die in Form von Gummis vorliegen können; Polysacchariden und/oder Oligosacchariden, die Galactose-Einheiten und/oder Galacturonsäure enthalten.

3. Verwendung nach Anspruch 1 oder 2, wonach das Ausgangsprodukt in Form einer wässrigen Stammlösung vorliegt.

4. Verwendung nach Anspruch 3, wonach das Ausgangsprodukt eine wässrige Stammlösung von Galacturonsäure ist.

5. Verfahren zur Herstellung von Schleimsäure, umfassend die folgenden Schritte:
(a) Bereitstellen einer wässrigen Stammlösung von Galacturonsäure,
(b) Kontaktieren der bei Schritt (a) bereitgestellten wässrigen Stammlösung von Galacturonsäure mit gasförmigem Ozon in einer Konzentration zwischen 150 und 220 g.m⁻³ TPN für eine Dauer von wenigstens 50 Minuten,
(c) Abtrennen der bei Schritt (b) gebildeten Schleimsäure.

6. Verfahren nach Anspruch 5, wonach die Konzentration an Galacturonsäure in der bei Schritt (a) bereitgestellten wässrigen Stammlösung wenigstens 8 Ma% Galacturonsäure, das heißt 80 kg Galacturonsäure pro 1000 kg Stammlösung beträgt.

7. Verfahren nach Anspruch 6, wonach die Dauer des Schrittes (b), des Kontaktierens mit Ozon, höchstens 60 Minuten beträgt.

8. Verfahren zur Herstellung von Schleimsäure nach einem der Ansprüche 5 bis 7, wonach nach Schritt (b) eine zweite Phase eines Kontaktierens der Reaktionslösung mit dem Ozon, in einer Konzentration zwischen 90 und 120 g.m⁻³ TPN, für eine Dauer zwischen 300 und 380 Minuten, vorzugsweise zwischen 320 und 360 Minuten vollzogen wird.

9. Verfahren zur Herstellung von Schleimsäure nach einem der Ansprüche 5 bis 8, wobei kein organisches Lösungsmittel verwendet wird.

10. Verfahren zur Herstellung von Schleimsäure nach einem der Ansprüche 5 bis 9, wobei kein homogener oder heterogener Katalysator während der verschiedenen Phasen der Reaktion verwendet wird.

11. Verfahren zur Herstellung von Schleimsäure nach einem der Ansprüche 5 bis 10, wobei das Ozon auf dem Trägergas reiner Sauerstoff erzeugt wird.

12. Verfahren zur Herstellung von Schleimsäure nach einem der Ansprüche 5 bis 11, wobei die in Form von Kristallen erhaltene Schleimsäure von der wässrigen Stammlösung im Laufe der Behandlung mit Ozon und/oder nach der Behandlung mittels eines Dekanters vom Typ Schnelldekanter abgetrennt wird.

13. Verfahren zur Herstellung von Schleimsäure nach einem der Ansprüche 5 bis 12, wobei die in Form von Kristallen erhaltene Schleimsäure mit reinem Wasser vom Typ Trinkwasser gewaschen wird.

14. Verfahren zur Herstellung von Schleimsäure nach Anspruch 13, wobei die gewaschenen Schleimsäurekristalle anschließend einer Trocknung mit trockener Heißluft unterzogen werden.

## Claims

1. The use of ozone pour synthesizing mucic acid from mono-, oligo- and/or poly-saccharides comprising galactose or galacturonic acid units.

2. The use according to claim 1, according to which the starting product comprises at least one from the group consisting of: galacturonic acid; a solution of pure galactose or containing impurities of the lactose type, dulcites, quercites, as well as various condensation or polymerization products which may appear as gums; polysaccharides and/or oligosaccharides containing galactose or galacturonic acid units.

3. The use according to claim 1 or 2, according to which the starting product is present as an aqueous mother solution.

4. The use according to claim 3, according to which the starting product is an aqueous mother solution of galacturonic acid.

5. A method for preparing mucic acid comprising the following steps:
(a) providing an aqueous mother solution of galacturonic acid;
(b) putting the aqueous mother solution of galacturonic acid provided in step (a) in contact with ozone gas at a concentration comprised between 150 and 220 g.m⁻³ (STP) for a period of at least 50 minutes;
(c) separating the mucic acid formed in step (b).

6. The method according to claim 5, according to which the concentration of galacturonic acid in the aqueous mother solution provided in step (a) is of at least 8% by mass of galacturonic acid, i.e. 80 kg of galacturonic acid for 1,000 kg of mother solution.

7. The method according to claim 6, according to which the duration of step (b), contacting with ozone, is of at most 60 minutes.

8. The method for preparing mucic acid according to any of claims 5 to 7, according to which, after step (b), a second phase is achieved for putting the reaction solution in contact with ozone at a concentration comprised between 90 and 120 g.m⁻³ (STP) for a period comprised between 300 and 380 minutes, preferentially between 320 and 360 minutes.

9. The method for preparing mucic acid according to any of claims 5 to 8, wherein no organic solvent is used.

10. The method for preparing mucic acid according to any of claims 5 to 9, wherein no homogeneous or heterogeneous catalyst is applied during the various phases the reaction.

11. The method for preparing mucic acid according to any of claims 5 to 10, wherein that the ozone is generated on the pure oxygen carrier gas.

12. The method for preparing mucic acid according to any of claims 5 to 11, wherein the mucic acid obtained as crystals is separated from the aqueous mother solution during treatment by ozone and/or after the treatment, by means of a decanter of the accelerated type.

13. The method for preparing mucic acid according to any of claims 5 to 12, wherein that the mucic acid obtained as crystals is washed with pure water of the drinking water type.

14. The method for preparing mucic acid according to claim 13, wherein that the washed crystals of mucic acid are then subject to drying in dry hot air.
